# FASCICULE DE BREVET EUROPEEN

(11) **EP 3 914 307 B1**
(45) Date de publication et mention de la délivrance du brevet: **28.09.2022**
(21) Numéro de dépôt: 20700951.5
(22) Date de dépôt: 23.01.2020
(51) Int. Cl.: A61L 27/38, A61L 27/56, A61L 27/60, G01N 33/50

(54) **SUBSTITUT DE TISSU CORPOREL**
ERSATZ FÜR KÖRPERGEWEBE
SUBSTITUTE FOR BODY TISSUE

(30) Priorité: 23.01.2019 FR 1900582
(43) Date de publication de la demande: 01.12.2021
(73) Titulaire: Microfactory, 75005 Paris (FR)
(72) Inventeur: MONTI, Fabrice, 91160 SAULX LES CHARTREUX (FR); FOUCHE, Florent, 75014 PARIS (FR)
(74) Mandataire: Regimbeau
(86) Numéro de dépôt international: PCT/EP2020/051656
(87) Numéro de publication internationale: WO 2020/152282

(56) Documents cités:
- WO-A1-2017/186423
- MORI NOBUHITO ET AL: "Skin integrated with perfusable vascular channels on a chip", BIOMATERIALS, ELSEVIER SCIENCE PUBLISHERS BV., BARKING, GB, vol. 116, 27 novembre 2016 (2016-11-27), pages 48-56, XP029845502, ISSN: 0142-9612, DOI: 10.1016/J.BIOMATERIALS.2016.11.031
- LINLIN HOU ET AL: "Artificial microfluidic skin for in vitro perspiration simulation and testing", LAB ON A CHIP, vol. 13, no. 10, 1 janvier 2013 (2013-01-01), page 1868, XP055328483, ISSN: 1473-0197, DOI: 10.1039/c3lc41231h

## Description

### DOMAINE DE L'INVENTION

La présente invention concerne le domaine des tissus corporels artificiels ou substituts de tissu corporel, notamment le domaine de la peau artificielle et/ou de la muqueuse de gencive artificielle, ainsi que le domaine de la cosmétique.

### ETAT DE LA TECHNIQUE

Les produits cosmétiques ou pharmacologiques peuvent nécessiter des tests sur la peau ou sur une muqueuse corporelle telle que la gencive avant leur commercialisation. Ces tests ont longtemps été réalisés sur des animaux. En vue de réduire la souffrance animale, des législations nationales ou régionales interdisent par exemple la mise sur le marché de produits cosmétiques dont la formulation a fait l'objet d'une expérimentation animale (directive 2003/15/CE et directive 76/768/CEE en Europe).

A cet effet, des substituts de peau ont été développés. Plus particulièrement, différents aspects de la peau ont été mimés.

WO 2017115056 décrit par exemple la fabrication d'un substitut de peau. Une encre biologique, comprenant des kératinocytes et des fibroblastes en suspension, est imprimée par fabrication additive sur une surface. L'encre biologique est gélifiée lors de la fabrication additive. Elle forme ainsi un substrat pour la culture des kératinocytes et des fibroblastes. Elle est ensuite incubée de manière à permettre la culture des kératinocytes et des fibroblastes dans le substrat formé par l'encre biologique gélifiée.

Toutefois, les tests de certains produits cosmétiques et pharmacologiques nécessitent que le substitut de tissu corporel présente des caractéristiques supplémentaires à la présence des cellules du tissu corporel à substituer. En particulier, le substitut de peau décrit par WO 2017115056 ne permet pas de tester des produits cosmétiques ou pharmacologiques interagissant avec la sueur, le sébum ou la salive.

A cet effet, WO 2017221870 décrit une méthode de fabrication d'un substitut de peau, le substitut de peau comprenant des follicules de cheveux et des glandes sébacées. Ainsi, le substitut de peau permet de mimer la production de sébum à la surface de la peau, ainsi que la présence de poils. Toutefois, la culture de glandes sébacées dans les cellules de peau peut s'avérer complexe et/ou onéreuse.

MORI NOBUHITO ET AL, "Skin integrated with perfusable vascular channels on a chip", BIOMATERIALS, ELSEVIER SCIENCE PUBLISHERS BV., BARKING, GB, vol. 116, 27 novembre 2016),pages 48-56 décrit l'utilisation d'une peau artificielle contenant une couche de kératinocyte et des canaux vasculaires perfusables pour des tests cosmétiques . Les cellules de la barrière cellulaire forment une jonction serrée.

Des tests *in vivo* sont toujours utilisés pour tester les produits cosmétiques ou pharmaceutiques anti-transpirants. Les produits anti-transpirants sont testés par gravimétrie sur des cobayes humains. Les produits anti-transpirants sont appliqués sur la peau des cobayes, qui sont ensuite placés dans un sauna. A la sortie du sauna, la sueur des cobayes est récoltée par des cotons pesés au préalable. Le pesage des cotons après la récolte de la sueur permet de mesurer un différentiel de masse et ainsi la production de sueur par les cobayes. Toutefois, cette méthode est imprécise. Il est par exemple impossible de détecter la différence d'efficacité entre un anti-transpirant comprenant 5 % de chlorure d'aluminium et un anti-transpirant comprenant 7 % de chlorure d'aluminium. Cette méthode ne permet pas non plus de détecter la différence d'efficacité entre des anti-transpirants comprenant des agents actifs différents à des concentrations égales. Enfin elle ne permet pas de mesurer les différents aspects physico-chimiques entraînant la formation des bouchons permettant de limiter la transpiration.

### EXPOSE DE L'INVENTION

Un but de l'invention est de proposer une solution pour mimer un tissu corporel de manière plus réaliste que le mimétisme obtenu grâce aux substituts de tissus corporels décrits dans l'art antérieur, notamment présentant des caractéristiques relatives à la production de sueur, à la production de sébum et/ou à la production de salive se rapprochant du comportement des tissus humains.

Ce but est atteint dans le cadre de la présente invention grâce à un substitut de tissu corporel choisi parmi de la peau artificielle et de la muqueuse artificielle, comprenant :
- un substrat comprenant une surface extérieure,
- une couche cellulaire comprenant une monocouche de cellules de peau ou de muqueuse, et
- au moins un pore s'étendant à travers la couche cellulaire,
dans lequel la couche cellulaire recouvre au moins une partie de la surface extérieure, et le substrat comprend une conduite propre à relier le pore à un système d'injection de liquide.

Comme le pore peut être relié à un système d'injection de liquide, il est possible de contrôler un débit de sueur, de sébum et/ou de salive arrivant par un pore formé par une couche de cellules du substitut de tissu corporel.

L'invention est avantageusement complétée par les caractéristiques suivantes, prises individuellement ou en l'une quelconque de leurs combinaisons techniquement possibles :
- la couche cellulaire comprend plusieurs monocouches,
- la couche cellulaire comprend une partie de couche cellulaire recouvrant une paroi interne de la conduite,
- le substitut de tissu corporel comprend un moyen de mesure de l'état physico-chimique d'un liquide dans la conduite,
- le substitut de tissu corporel comprend un moyen de mesure du débit d'un liquide dans la conduite,
- le substitut de tissu corporel comprend un moyen de mesure de l'état métabolique et/ou de l'état physico-chimique de la couche cellulaire,
- le substitut de tissu corporel comprend un système d'injection de liquide, le système d'injection de liquide comprenant :
   au moins un réservoir apte à contenir le liquide et une phase gazeuse séparés par une interface à une hauteur h₁, le réservoir étant relié fluidiquement à la conduite,
   un actuateur de pression configuré pour imposer une pression de la phase gazeuse à l'intérieur du réservoir,
   un débitmètre,
   un régulateur par asservissement en boucle fermée,
   le débitmètre étant configuré pour mesurer le débit du liquide dans la conduite et pour transmettre une valeur mesurée du débit au régulateur, le régulateur étant configuré pour transmettre une commande de régulation à l'actuateur de pression et le pore étant agencé à une hauteur h₂ différente de hi,
- h₂ est strictement supérieure à h₁ et l'actuateur de pression est configuré pour imposer une pression supérieure à la pression atmosphérique dans le réservoir,
- les cellules sont choisies au moins parmi des cellules du derme, des cellules de l'épiderme, des cellules de l'hypoderme, des cellules de la jonction dermo-épidermique, des kératinocytes tels que des kératinocytes de peau et des kératinocytes de gencive, des mélanocytes et leurs combinaisons,
- les cellules sont choisies au moins parmi des cellules de l'épiderme, et la couche cellulaire forme une couche de cellules stratifiées,
- les cellules de l'épiderme sont différentiées les unes par rapport aux autres,
- le substitut de tissu corporel comprend au moins un tensioactif adsorbé sur la surface extérieure du substrat, entre la surface extérieure et la couche cellulaire,
- le tensioactif est choisi au moins parmi du collagène, de la fibronectine, de la laminine,
- le liquide est choisi au moins parmi de la sueur, de la sueur artificielle, du sébum, du sébum artificiel, de la salive, de la salive artificielle et leurs combinaisons,
- le pore présente un diamètre compris entre 0,5 µm et 3 mm, notamment compris entre 1 µm et 100 µm, et préférentiellement compris entre 3 µm et 80 µm.

Un autre objet de l'invention est l'utilisation d'un substitut de tissu corporel selon un mode de réalisation de l'invention, pour mesurer l'efficacité d'un déodorant.

Un autre objet de l'invention est l'utilisation d'un substitut de tissu corporel selon un mode de réalisation de l'invention, pour mesurer l'effet de la sueur sur un produit cosmétique recouvrant la couche cellulaire et le pore.

Un autre objet de l'invention est l'utilisation d'un substitut de tissu corporel selon un mode de réalisation de l'invention, pour mesurer l'effet du sébum sur un produit cosmétique recouvrant la couche cellulaire et le pore.

Un autre objet de l'invention est l'utilisation d'un substitut de tissu corporel selon un mode de réalisation de l'invention, pour mesurer la perméabilité d'un vernis recouvrant la couche cellulaire et le pore.

Un autre objet de l'invention est un procédé de fabrication d'un substitut de tissu corporel selon un mode de réalisation de l'invention, comprenant les étapes de :
- fourniture d'un substrat comprenant une surface extérieure, et une conduite propre à relier fluidiquement la surface extérieure à un système d'injection de liquide,
- culture de cellules de peau ou de muqueuse sur la surface extérieure pendant un temps prédéterminé permettant de former une couche cellulaire comprenant une monocouche de cellules de peau ou de muqueuse recouvrant au moins une partie de la face extérieure et comprenant également un pore.

### DESCRIPTION DES FIGURES

D'autres caractéristiques, buts et avantages de l'invention ressortiront de la description qui suit, qui est purement illustrative et non limitative, et qui doit être lue en regard des dessins annexés sur lesquels :
[Fig. 1] La figure 1 illustre schématiquement une partie d'un substitut de tissu corporel selon un mode de réalisation de l'invention.
[Fig. 2] La figure 2 illustre schématiquement une partie d'un substitut de tissu corporel selon un mode de réalisation de l'invention.
[Fig. 3] La figure 3 illustre schématiquement un substitut de tissu corporel selon un mode de réalisation de l'invention.
[Fig. 4] La figure 4 illustre schématiquement un substitut de tissu corporel selon un mode de réalisation de l'invention.
[Fig. 5] La figure 5 illustre schématiquement un procédé de fabrication d'un substitut de tissu corporel.
[Fig. 6] La figure 6 illustre schématiquement la prolifération de kératinocytes sur une surface extérieure de PDMS.
[Fig. 7] La figure 7 illustre schématiquement la prolifération de cellules sur une surface extérieure de PDMS sur laquelle des tensioactifs sont adsorbés.

Sur l'ensemble des figures, les éléments similaires portent des références identiques.

### DEFINITIONS

On entend par « monocouche de cellule » un assemblage compact de cellules formant une couche ne comprenant pas plus d'une cellule dans son épaisseur, et dans lequel la densité de surface de cellules est supérieure à 0,9, et préférentiellement supérieure à 0,95. Une monocouche de cellules comprend au moins 1000 cellules, préférentiellement au moins 10000 cellules.

On entend par « tensioactif » tout composé modifiant la tension superficielle d'une surface, et préférentiellement augmentant l'énergie de la surface. Préférentiellement, le tensioactif peut être une protéine.

On entend par « pore » une ouverture s'étendant à travers la couche de cellules.

On entend par « diamètre » d'un pore la dimension maximale d'un pore mesurée dans un plan parallèle à la couche de cellules. Le diamètre peut être mesuré par des méthodes de microscopie typiques, par exemple en imageant un pore par microscopie inversée, préférentiellement en utilisant un microscope à contraste interférentiel. Il est également possible de marquer les cellules du pore, et d'observer le ou les pores par microscopie en fluorescence.

On entend par « sueur naturelle » un liquide biologique sécrété par les glandes sudorales lors de la transpiration. La sueur naturelle est, par exemple, préalablement prélevée à partir d'un humain. La sueur naturelle est produite par des glandes eccrines et/ou des glandes apocrines.

On entend par « sueur artificielle » une solution aqueuse comprenant du chlorure de sodium (NaCl), de l'acide lactique, de l'urée, de la BSA (acronyme anglais de *bovine sérum albumin*). La sueur artificielle peut également comprendre de l'ammoniac de manière à tamponner ladite sueur artificielle à un pH prédéterminé, de manière à mimer les différents types de sueurs selon leur pH. Le pH de ladite sueur artificielle peut être préférentiellement compris entre 3,8 et 7.

On entend par « sébum naturel » un liquide biologique sécrété par les glandes sébacées. Le sébum naturel est, par exemple, préalablement prélevé à partir d'un humain. Le sébum naturel est le liquide biologique produit par les glandes sébacées de la peau.

On entend par « sébum artificiel » un liquide adapté à former un film lipidique sur une surface de cellules. Préférentiellement, le sébum artificiel comprend au moins un élément choisi parmi du squalène, un triglycéride d'acide gras linéaire, un ester d'acide gras linéaire, un alcool gras linéaire, un ester et du cholestérol.

On entend par « salive naturelle » un liquide biologique sécrété par les glandes salivaires. La salive naturelle est, par exemple, préalablement prélevée à partir d'un humain.

On entend par « salive artificielle » une solution aqueuse permettant de mimer la salive naturelle. La salive artificielle comprend des ions sodium, potassium, chlorure et bicarbonate. La salive artificielle comprend préférentiellement au moins un élément choisi parmi de l'urée, du glucose, de l'acide urique, de l'acide citrique, des acides aminés, de la créatinine, du cholestérol, des phospholipides, de l'ARN et des cellules.

On entend par « vernis » un produit cosmétique sec comprenant une résine sèche, et préférentiellement au moins un élément choisi parmi un agent filmogène, un agent plastifiant, des traces de solvants, des pigments, et un agent thixotrope.

### DESCRIPTION DETAILLEE DE L'INVENTION

### Principes généraux

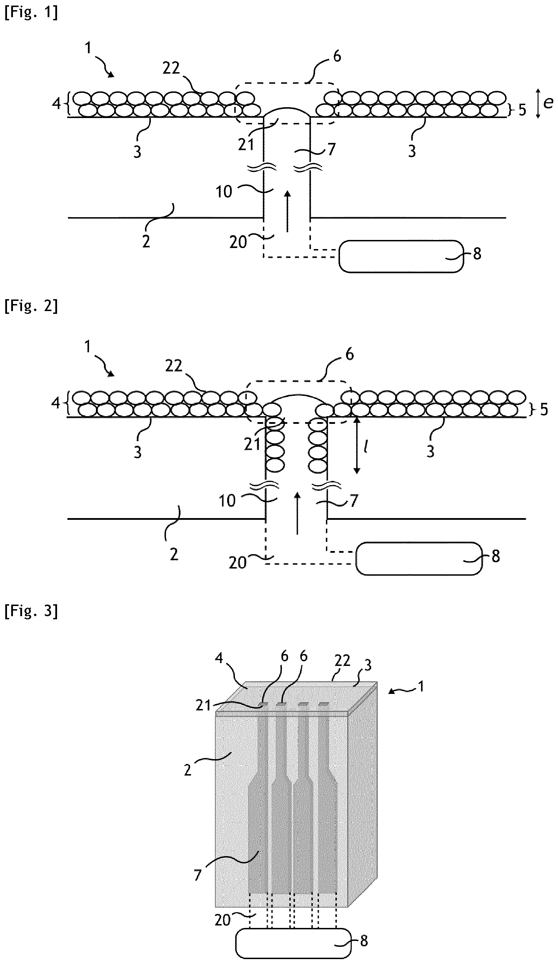

En référence à la figure 1, le substitut 1 de tissu corporel comprend un substrat 2. Le substrat 2 comprend une surface extérieure 3. La surface extérieure 3 peut être préférentiellement plane. Par « surface extérieure », on entend dans l'invention une surface en périphérie du substrat 2, dont le contact est préférentiellement accessible pour un autre élément venant à la rencontre du substrat 2. Le substitut 1 comprend au moins une conduite 7. La conduite 7 peut traverser le substrat 2, et déboucher sur la surface extérieure 3 par un orifice 21.

Le substitut 1 de tissu corporel comprend également une couche cellulaire 4, comprenant au moins une monocouche 5 de cellules de peau et/ou de muqueuse, et un pore 6 s'étendant à travers la couche cellulaire 4. La couche cellulaire 4 recouvre au moins en partie la face extérieure 3 du substrat 2. La couche cellulaire 4 présente également une face cellulaire extérieure 22 (ou deuxième face 22 ou surface libre 22 au sens où elle n'est pas directement en contact avec un autre élément du système), à l'opposé de la face de la couche cellulaire 4 en contact avec le substrat 2 (ou première face). L'épaisseur e de la couche cellulaire 4 est définie entre la première face et la deuxième face. Ainsi, la première face et la deuxième face 22 s'étendent transversalement à l'épaisseur e de la couche cellulaire 4.

La couche cellulaire 4 étant une monocouche ou une superposition de plusieurs couches de cellules, l'épaisseur e de la couche cellulaire 4 entre la surface extérieure 3 du substrat 2 et la face cellulaire 22 extérieure est préférentiellement comprise entre 2 µm et 3 mm, notamment entre 10 µm et 1 mm, et plus préférentiellement entre 30 µm et 130 µm. On entend par « épaisseur » le sens usuel du terme, c'est-à-dire la plus petite dimension de la couche, séparant deux surfaces. L'épaisseur e de la couche cellulaire 4 est ainsi dans une direction transverse à la tangente de la face extérieure 3 du substrat 2. Ainsi, il est possible de paralléliser un grand nombre de pores 6 sur les deux dimensions de la deuxième face 22, chaque pore 6 traversant une épaisseur e de peau réduite. Il est de plus possible de limiter les variations du diamètre du pore 6, entraînées par les variations de conditions physico-chimique du pore 6 (variation saline, température, etc).

Le substitut 1 de tissu corporel comprend préférentiellement un système 8 d'injection de liquide 10. La conduite 7 est propre à relier le pore 6 au système 8 d'injection de liquide 10. Ainsi, il est possible d'acheminer un liquide corporel et/ou un liquide mimant un liquide corporel à un débit contrôlé jusqu'au pore 6 de la couche cellulaire 4, tout en supportant mécaniquement la couche cellulaire 4 et en présentant une face cellulaire extérieure 22 du substitut de tissu corporel formée de cellules de peau et/ou de muqueuse et d'au moins un pore apte à émettre le liquide 10. En d'autres termes, le pore 6 débouche sur la deuxième face 22 libre de la couche cellulaire 4, de sorte à mimer un pore de la peau ou d'une muqueuse. La conduite 7 est préférentiellement reliée fluidiquement au système 8 d'injection par un canal de liaison 20. Le canal de liaison peut par exemple être un tube flexible, raccordé de manière étanche à ses extrémités respectivement au substrat 2 et au système 8 d'injection. Le canal de liaison 20 peut également être formé par un tube raccordé de manière étanche respectivement au système 8 d'injection et à un passage d'un support de substrat 2, le passage du support étant relié de manière étanche à ses extrémités au tube et à la conduite 7.

Préférentiellement, le pore 6 s'étendant à travers la couche cellulaire 4 présente un diamètre compris entre 0,5 µm et 3 mm, notamment compris entre 3 µm et 100 µm, et préférentiellement compris entre 3 µm et 80 µm. Ainsi, le pore 6 mime structurellement un pore naturel de la peau et/ou d'une muqueuse. En effet, un pore 6 de la peau humaine présente typiquement un diamètre compris entre 3 µm et 3 mm. Un pore de gencive humaine peut présenter un diamètre inférieur à celui d'un pore de peau humaine, typiquement compris entre 1 µm et 100 µm. Cette variabilité du diamètre de pore est dépendante de la partie muqueuse et/ou de la peau considérée et de la personne considérée.

Le substrat 2 supporte la couche cellulaire 4. Préférentiellement, l'orifice 21 de la conduite 7 coïncide avec le pore 6 de la couche cellulaire 4. Dans un mode de réalisation de l'invention, le pore 6 peut être en vis-à-vis direct de l'orifice 21 de manière à coïncider avec ledit orifice 21.

Le diamètre de l'orifice 21 (à l'endroit où la conduite 7 est reliée au pore 6) est préférentiellement compris entre 1/3 et 3 fois le diamètre du pore 6, notamment compris entre ½ et 2 fois le diamètre du pore 6, et plus préférentiellement égale au diamètre du pore 6. Ainsi, la couche cellulaire 4 est supportée mécaniquement par le substrat 2 autour du pore 6, tout en permettant de présenter une face cellulaire extérieure 22 du substitut 1 de tissu corporel majoritairement formée par des cellules.

En référence à la figure 2, la couche cellulaire 4 comprend une partie de couche cellulaire 4 recouvrant une paroi interne de la conduite 7. Ainsi, le pore 6 peut mimer les variations physico-chimiques ou structurelles d'un pore naturel lors de la mise en contact avec un liquide tel que la sueur, le sébum ou la salive. En effet, les variations par exemple de pH ou de stringence peuvent entraîner des variations d'épaisseur de la couche cellulaire 4 et ainsi modifier le diamètre du pore 6. Préférentiellement, la longueur *l* de la partie de couche cellulaire s'étendant dans la conduite 7 à partir du pore 6 est inférieure à 2 mm, notamment inférieure à 500 µm, et plus préférentiellement inférieure à 100 µm. Ainsi, il est possible d'éviter la contamination biologique des réservoirs de liquide mimant un liquide biologique.

### Cellules et liquides du substitut de tissu corporel

Les cellules utilisées pour former la couche cellulaire 4 comprennent des cellules choisies au moins parmi des cellules du derme, des cellules de l'épiderme, des cellules de l'hypoderme, des cellules de la jonction dermo-épidermique, des kératinocytes tels que des kératinocytes de peau et des kératinocytes de gencive, des mélanocytes, des cellules de Langerhans, des cellules de Merkel et leurs combinaisons. Ainsi, il est possible de mimer les pores de la peau ou des gencives.

Préférentiellement, les cellules sont choisies parmi les cellules de l'épiderme, de manière à mimer la peau. La couche cellulaire 4 peut par exemple former une couche de cellules stratifiées. Le substrat 2 peut par exemple jouer le rôle de membrane basale, et peut être recouvert d'une stratification de couches additionnelles choisies parmi une couche basale, une couche épineuse, une couche granuleuse et une couche cornée. La surface extérieure 3 du substrat 2 est ainsi recouverte d'une monocouche 5 de cellules, éventuellement recouverte elle-même d'une stratification. La couche cellulaire 4 peut préférentiellement comprendre des cellules différentiées les unes par rapport aux autres. Une couche épineuse et/ou une couche basale de la couche de cellules 4 peut par exemple comprendre des kératinocytes différentiés par rapport aux kératinocytes de la couche basale. Ainsi, il est possible de mimer la structure des différentes couches de la peau sur le substrat 2.

Le liquide 10 peut être choisi parmi de la sueur, de la sueur artificielle, du sébum, du sébum artificiel, de la salive, de la salive artificielle et leurs combinaisons. Préférentiellement, on utilise des couples couche cellulaire 4/liquide 10 permettant de mimer le tissu corporel à substituer.

Le substitut 1 de tissu corporel peut préférentiellement comprendre un moyen de mesure de l'état métabolique de la couche cellulaire 4 et/ou de l'état physico-chimique de la couche cellulaire 4. Ainsi, il est possible de vérifier la pertinence de l'utilisation du substitut 1 au regard de la couche cellulaire 4. Le moyen de mesure de l'état métabolique de la couche cellulaire 4 et/ou de l'état physico-chimique de la couche cellulaire 4 peut être choisi, par exemple, parmi un moyen de mesure de l'impédance de la couche cellulaire 4 (par exemple de l'impédance entre deux électrodes déposées sur la surface extérieure 3), et un moyen de mesure de la fluorescence des cellules, préalablement marquées par un fluorophore (par exemple le DAPI).

### Substrat 2

En référence à la figure 3, le substitut 1 comprend un réseau formé d'une pluralité de pores 6. Le réseau peut être un réseau unidimensionnel, comme illustré en figure 3. Le réseau peut également être un réseau bidimensionnel. Alternativement, les différents pores 6 peuvent être répartis aléatoirement sur la surface extérieure 3 de manière à mimer les pores de la peau. Dans ce cas, la densité de surface des pores 6, c'est-à-dire le rapport entre le nombre de pores 6 et l'aire totale de la face cellulaire extérieure 22 en cm², peut être préférentiellement comprise entre 10 et 1000. Ainsi, la densité de surface des pores 6 mime celle de la peau naturelle.

Le substitut 1 peut également comprendre un réseau de conduites 7. Chaque conduite 7 est reliée à l'une de ses extrémités à l'un des orifices 21 ménagés sur la surface extérieure 3 du substrat 2. Toutes ou une partie des conduites 7 peuvent être raccordées à un orifice 21 ou à plusieurs orifices 21. Préférentiellement, le substrat 2 peut être une puce microfluidique. Les conduites 7 peuvent également présenter, à une extrémité opposée à celle qui est reliée à l'orifice 21, une entrée fluidique adaptée à être reliée au système 8 d'injection.

Le substrat 2 peut être réalisé en partie en un premier matériau, et préférentiellement entièrement réalisé en ce premier matériau. Le substrat 2 peut comprendre deux couches superposées réalisées chacune en premier matériau et collées entre elles. Le substrat 2 peut par exemple comprendre deux couches en PDMS. L'une des couches peut être moulée ou micro-usinée de manière à présenter sur l'une de ses faces des gorges propres à former une partie de la ou des conduites 7, une fois que les deux couches sont superposées. L'agencement des gorges sur la face de la couche est prévu pour que la ou les conduites 7 débouchent sur une bordure de ladite face. Ainsi, lors de la superposition des deux couches, le ou les orifices 21 sont formés sur une face latérale s'étendant transversalement à l'interface entre les deux couches, cette face latérale correspondant à la surface extérieure 3. Dans un autre mode de réalisation, compatible avec le mode de réalisation précédent, on peut couper l'empilement formé par la superposition de couches au niveau de la ou des conduites 7 pour former à la fois la surface extérieure 3 et la ou les orifices 21 débouchant sur la surface extérieure 3.

Le premier matériau peut être choisi parmi un copolymère d'oléfine cyclique, du polyméthacrylate de méthyle, du polystyrène, du polycarbonate et du polyéthylène glycol diacrylate. Ainsi, le ou les canaux peuvent résister chimiquement à des solvants tels que la silicone, des alcools, des huiles naturelles ou artificielles, et/ou des solutions comportant des micro ou nanoparticules et/ou des capsules. Le premier matériau peut également être choisi parmi un gel d'agarose, ou tout autre matériau biocompatible.

Le premier matériau ou l'un des matériaux du substrat 2 peut être préférentiellement transparent aux longueurs d'ondes de la lumière visible (c'est-à-dire dans une gamme de longueurs d'ondes comprises entre 380 à 780 nanomètres).

Le substitut 1 peut faire partie d'un ensemble comprenant un système d'imagerie 19 configuré pour acquérir des images des conduites 7 ou d'une partie des conduites 7 au travers du substrat 2 transparent, et/ou une partie de la surface extérieure 3 comprenant le ou les pores 6. Ainsi, il est possible d'observer par imagerie les modifications physico-chimiques se produisant dans la ou les conduites 7, et préférentiellement autour du ou des pores 6.

### Système 8 d'injection

Le débit de liquide 10 s'écoulant à travers le pore 6 est un paramètre important pour mimer un tissu biologique naturel. Par exemple, la surface de la peau naturelle comprend des pores présentant un ménisque de sueur ou de sébum. Le niveau de ce ménisque peut être quasi-constant, par l'effet combiné du débit de sueur et/ou de sébum arrivant des glandes de production jusqu'au pore, et de l'évaporation de la sueur et/ou du sébum.

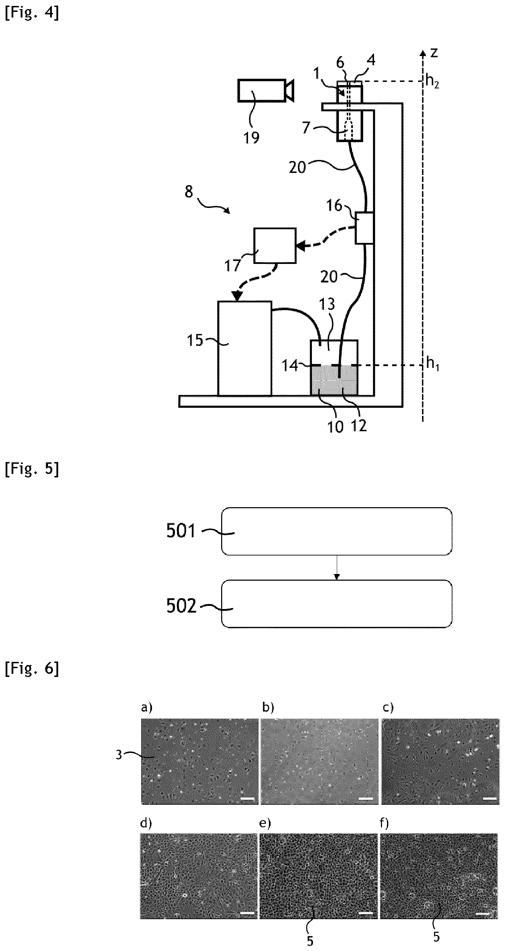

En référence à la figure 4, le substitut 1 de tissu corporel peut comprendre un système 8 d'injection.

Le système 8 d'injection peut comprendre un moyen de mesure du débit d'un liquide dans la conduite 7. Ce moyen de mesure du débit peut par exemple être un débitmètre 16. Le débitmètre 16 peut être agencé le long du canal de liaison 20. Le moyen de mesure du débit est adapté pour mesurer le sens de l'écoulement en transmettant une valeur positive ou négative du débit mesuré. Le débit est préférentiellement mesuré entre -60 µL/min et 60 µL/min.

Le système 8 d'injection peut comprendre un réservoir 12 apte à contenir le liquide 10 et une phase gazeuse 13 séparés par une interface 14 s'étendant à une hauteur h₁. Le réservoir 12 est relié fluidiquement à la conduite 7. Typiquement, le réservoir 12 peut contenir jusqu'à 100 mL de liquide 10.

Le système 8 d'injection peut également comprendre un actuateur de pression 15, aussi appelé contrôleur de pression, apte à imposer une pression à la phase gazeuse contenue dans le réservoir 12. L'actuateur de pression 15 peut comporter une pluralité de sorties dans lesquelles les pressions imposées sont indépendantes et/ou couplées. Une sortie de l'actuateur de pression 15 est reliée à un tuyau d'imposition de la pression, lui-même relié à la phase gazeuse 13 du réservoir 12. Si la pression imposée est supérieure à la pression atmosphérique, la surpression correspondante peut être comprise entre 0 bar et 20 bar et préférentiellement comprise entre 0 bar et 2 bar. Typiquement, une variation de pression peut être imposée dans la phase gazeuse 13 par l'actuateur de pression 15 en moins de 200 ms, préférentiellement en moins de 100 ms et préférentiellement en moins de 50 ms.

Un canal de liaison 20 est relié au réservoir 12 *via* une ouverture du réservoir 12 permettant d'agencer une extrémité du canal de liaison 21 dans le liquide 10.

Le canal de liaison 20 relie le réservoir 12 à la conduite 7. Par canal de liaison 20, on entend un canal, un tuyau ou une tubulure, par exemple souple, permettant de relier le réservoir 12 à la conduite 7. On entend également par canal de liaison 20 toute succession de canaux reliés en série permettant de relier le réservoir 12 à la conduite 7, par exemple une tubulure reliée à un manchon métallique, à un passage formé par le support, ou au canal d'un instrument de mesure lui-même relié à une autre tubulure.

L'ensemble constitué du substrat 2 et de la couche de cellules 4 est agencé, par rapport aux autres éléments du substitut 1, de sorte que le pore 6 est situé à une hauteur h₂ strictement différente de la hauteur h₁ définie précédemment. Par exemple, la différence de hauteur Δh entre h₂ et h₁ peut être comprise entre 1 cm et 3 m, préférentiellement entre 5 cm et 1 m et préférentiellement entre 10 cm et 50 cm.

En imposant une pression supérieure à la pression atmosphérique dans la phase gazeuse 13 du réservoir 12, on peut injecter le liquide 10 du réservoir 12 dans le canal de liaison 21 puis dans la conduite 7.

Ainsi, une particule de liquide 10, à la hauteur h₂ d'un pore 6, subit au moins deux forces : une force due à la pression imposée par l'actuateur de pression 15, entraînant une particule de fluide vers l'extérieur de la conduite 7 dans le cas d'une pression imposée supérieure à la pression atmosphérique, et une force hydrostatique, entraînant la particule de liquide 10 vers l'intérieur de la conduite 7 dans le cas d'une valeur de la différence de hauteur Δh positive.

Théoriquement, la pression d'équilibrage ΔPₛₜₒₚ imposée par l'actuateur de pression 15 peut être exprimée sous la forme ΔPₛₜₒₚ = ρ.g.Δh, ou p correspond à la densité volumique du liquide 10, et g correspond à la valeur absolue de l'accélération de pesanteur.

Le système 8 d'injection peut également comprendre un régulateur 17 par asservissement en boucle fermée. Le régulateur 17 peut comprendre un ordinateur ou un circuit électronique. Le débitmètre 16 est configuré pour transmettre une valeur mesurée du débit de la conduite 7 au régulateur 17. Le régulateur 17 est configuré pour transmettre une commande de régulation à l'actuateur de pression 15, en fonction de la valeur mesurée du débit de la conduite 7.

Ainsi, une boucle d'asservissement fermée peut être formée pour contrôler précisément le débit dans la conduite 7.

L'utilisateur peut par exemple donner une consigne de débit au régulateur 17. Une consigne de débit nul peut être donnée pour maintenir de manière précise un ménisque de liquide 10 à la hauteur h₂ d'un pore 6. Une régulation du débit de la conduite 7 en boucle fermée est réalisée par l'action de la force de pression imposée au liquide 10 et à la rétroaction de la force hydrostatique imposée au liquide 10. Avantageusement, la régulateur 17 est un régulateur de type P.I.D. (acronyme de « Proportionnel, Intégral et Dérivée »). Une boucle d'asservissement peut être réalisée de la manière suivante : le débitmètre 16 transmet une information représentative du débit de la conduite 7 au régulateur 17. Si le débit est positif, le régulateur 17 commande l'actuateur de pression 15 de manière à diminuer la pression imposée dans la phase gazeuse 13 du réservoir 12. Au contraire, si le débit mesuré est négatif, le régulateur 17 commande l'actuateur de pression 15 de manière à augmenter la pression imposée dans la phase gazeuse 13 du réservoir 17. Cette opération peut être répétée de manière automatique un grand nombre de fois (par exemple plus de dix fois) de manière à converger vers un débit nul ou suffisamment proche de zéro. Par « suffisamment proche de zéro », on entend que la valeur absolue du débit moyen, mesurée par exemple comme une moyenne glissante sur une seconde, est inférieure ou égale à un seuil prédéterminé, par exemple 0,2 µL/min, notamment inférieure ou égale à 0,1 µL/min et plus préférentiellement inférieure ou égale à 0,05 µL/min.

Les différentes conduites 7 du substrat 2 peuvent présenter des différences structurelles qui entraînent une résistance hydrodynamique différente entre les différentes conduites 7. Préférentiellement, une pluralité d'asservissements en boucle fermée peut être mis en œuvre indépendamment pour contrôler le débit de chacune des conduites 7. Ainsi, il est possible de commander un débit égal dans chacune des conduites 7 du substitut 1.

En référence à la figure 5, un aspect de l'invention concerne un procédé de fabrication du substitut 1 de tissu corporel. Le procédé comprend les étapes de :
- fourniture (étape 501) d'un substrat 2 comprenant une surface extérieure 3, et une conduite 7 propre à relier fluidiquement la surface extérieure 3 à un système d'injection de liquide 8,
- culture (étape 502) de cellules de peau ou de muqueuse sur la surface extérieure 3 pendant un temps prédéterminé permettant de former une couche cellulaire 4 comprenant une monocouche 5 de cellules de peau ou de muqueuse recouvrant au moins une partie de la face extérieure 3, et un pore 6 s'étendant à travers la couche cellulaire 4.

En référence à la figure 6, la surface extérieure 3 peut être cellularisée, c'est-à-dire recouverte au moins d'une monocouche 5 de cellules. La figure 6 comprend des microphotographies d'une partie d'une surface extérieure 3 en PDMS, acquises par un microscope à contraste interférentiel. La surface extérieure 3, dont l'aire est dans l'exemple de la figure 6 égale à 3 cm², est ensemencée par 20000 kératinocytes. La partie a) de la figure 6 illustre la surface extérieure 3 recouverte de kératinocytes 30 minutes après l'ensemencement, la partie b) de la figure 6 illustre la surface extérieure 3 recouverte de kératinocytes 2 heures après l'ensemencement, la partie c) de la figure 6 illustre la surface extérieure 3 recouverte de kératinocytes 24 heures après l'ensemencement, la partie d) de la figure 6 illustre la surface extérieure 3 recouverte de kératinocytes 3 jours après l'ensemencement, la partie e) de la figure 6 illustre la surface extérieure 3 recouverte de kératinocytes 5 jours après l'ensemencement et la partie f) de la figure 6 illustre la surface extérieure 3 recouverte de kératinocytes 7 jours après l'ensemencement.

La surface extérieure 3 peut être recouverte d'une monocouche 5 de cellules 5 jours après l'ensemencement. Il est ainsi possible de mesurer la durée minimale nécessaire de l'étape 502 de culture permettant de former une couche cellulaire 4 comprenant une monocouche 5 de cellules de peau ou de muqueuse recouvrant au moins une partie de la surface extérieure 3.

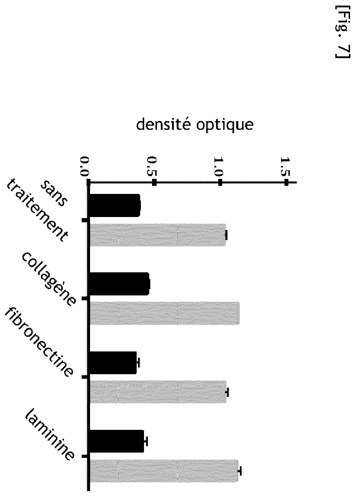

En référence à la figure 7, le substitut 1 de tissu corporel peut comprendre au moins un tensioactif 18 adsorbé sur la surface extérieure 3 du substrat 2, entre la surface extérieure 3 et la couche cellulaire 4. Ainsi, la prolifération et/ou la différentiation des cellules peut être optimisée sur la surface extérieure 3. De préférence, le tensioactif 18 est choisi au moins parmi du collagène, de la fibronectine, de la laminine et leurs combinaisons. La figure 7 illustre une mesure de la densité optique, correspondant à la densité de cellules sur la surface extérieure 3, pour différents tensioactifs 18 adsorbés sur une surface extérieure 3 en PDMS. Les colonnes en noir correspondent à un ensemencement initial de 5000 cellules sur une surface de 3 cm² de PDMS et les colonnes grises correspondent à un ensemencement initial de 20000 cellules sur une surface de 3 cm² de PDMS. La densité optique est mesurée 5 jours après l'ensemencement des cellules. Trois tensioactifs différents, adsorbés sur la surface de PDMS sont testés : le collagène, la fibronectine et la laminine 332. Préférentiellement, le substitut 1 de tissu corporel comprend du collagène adsorbé sur la surface extérieure 3. Il a en effet été découvert que le collagène permet une meilleure adhésion des cellules à la surface extérieure 3 en comparaison à la fibronectine et à la laminine. Ainsi, la prolifération et/ou la différentiation des cellules est optimisée quand le substitut 1 de tissu corporel comprend du collagène adsorbé sur la surface extérieure 3 du substrat 2, en comparaison avec à de la laminine ou à de la fibronectine adsorbée sur la surface extérieure 3.

### Utilisation du substitut pour tester l'efficacité d'un produit déodorant

Un aspect de l'invention est l'utilisation du substitut 1 de tissu corporel pour mesurer l'efficacité d'un déodorant.

La face cellulaire extérieure 22 de la couche cellulaire 4 peut recevoir un produit déodorant. Cette face peut être vue comme une modélisation de la peau pour l'évaluation d'un produit déodorant. Différentes formes galéniques du déodorant peuvent être choisies : un gel, une crème, une émulsion, une mousse et/ou une solution anhydre. Ainsi, il est possible d'évaluer d'autres formes galéniques d'un produit déodorant que la forme liquide. Une couche de produit déodorant peut être déposée sur la face cellulaire extérieure 22. Cette couche de déodorant recouvre au moins un pore 6, et préférentiellement l'ensemble des pores 6 du substitut 1.

Un capteur peut être avantageusement agencé sur une surface du substrat 2, en particulier sur une paroi de la conduite 7 et/ou sur la surface extérieure 3. Le capteur peut permettre de déterminer un paramètre physico chimique ou biologique avant, après ou lors d'une réaction entre la sueur et un produit déodorant. Avantageusement, le capteur peut être apte à mesurer un pH, une présence ou un type d'acides aminés, une présence ou un type de nucléotides, une enzyme et/ou une bactérie ou une population de bactéries. Sans introduire de limitation sur le type de capteur utilisé, le capteur peut être capacitif, électrochimique, résistif et être implanté dans le substitut 1 par microfabrication d'une électrode métallique. Le capteur peut permettre de caractériser les propriétés antibactériennes du produit déodorant, qui peut être dépourvu d'action anti-transpirante, c'est-à-dire ne conduisant pas à la formation d'un bouchon et/ou de particules formées par floculation. Le capteur peut également être un capteur mécanique et/ou optique. On peut par exemple utiliser le système d'imagerie 19.

Préférentiellement, le procédé d'évaluation de l'efficacité d'un produit déodorant peut comprendre les étapes de :
- introduction de la sueur naturelle ou artificielle dans toute la conduite 7 en contrôlant l'actuateur de pression 15 de manière à imposer une pression dans la phase gazeuse du réservoir 12 comportant de la sueur,
- mesure d'un débit de sueur dans la conduite 7, transmission de la mesure du débit au régulateur 17, le régulateur 17 imposant une consigne de pression à l'actuateur de pression 15, de manière à imposer un débit sensiblement nul dans la conduite 7 par asservissement en boucle fermée ;
- mise en contact la sueur et du produit déodorant à tester à travers le pore 6 et attente pendant le déroulement d'une réaction entre la sueur et le produit déodorant ;
- détermination d'au moins un paramètre choisi parmi un paramètre physico-chimique et un paramètre biologique caractéristique de ladite réaction entre la sueur et du déodorant de manière à évaluer l'efficacité du déodorant.

Préférentiellement, lors de l'étape d'attente, on attend la formation d'un bouchon produit par la réaction entre le déodorant et la sueur.

L'étape de détermination d'au moins un paramètre peut comprendre le contrôle de l'actuateur de pression 15 de manière à augmenter la pression dans la phase gazeuse 13 jusqu'à l'augmentation du débit dans la conduite 7 au-delà d'un seuil de débit prédéterminé. Ainsi, il est possible de détecter facilement la présence d'un bouchon formé dans la conduite 7 entre la sueur et le déodorant, et/ou de mesurer sa taille, en cassant ce bouchon par une augmentation de la pression de liquide 10 entraînée par l'augmentation de la pression dans la phase gazeuse 13.

### Utilisation du substitut pour mesurer l'effet du sébum et/ou de la sueur sur un produit cosmétique

Un procédé de mesure de l'effet du sébum et/ou de la sueur sur un produit cosmétique selon un aspect de l'invention peut comprendre les étapes de :
- introduction de la sueur naturelle ou artificielle et/ de sébum dans toute la conduite 7 en contrôlant l'actuateur de pression 15 de manière à imposer une pression dans la phase gazeuse du réservoir 12 comportant de la sueur et/ou du sébum,
- mesure d'un débit de sueur et/ou de sébum dans la conduite 7, transmission de la mesure du débit au régulateur 17, le régulateur 17 imposant une consigne de pression à l'actuateur de pression 15, de manière à imposer un débit sensiblement nul dans la conduite 7 par asservissement en boucle fermée ;
- mise en contact la sueur et/ou du sébum et du produit cosmétique à tester à travers le pore 6 et attente pendant le déroulement d'une réaction entre d'une part la sueur et/ou le sébum et d'autre part le produit cosmétique ;
- détermination d'au moins un paramètre choisi parmi un paramètre physico-chimique et un paramètre biologique caractéristique de ladite réaction entre d'une part la sueur et/ou le sébum et d'autre part le produit cosmétique de manière à évaluer l'effet du sébum et/ou de la sueur sur le produit cosmétique.

Le produit cosmétique peut être préférentiellement choisi au moins parmi un produit de maquillage, une crème solaire, une crème hydratante, un fond de teint, un rouge à lèvre, un ligneur (ou « *eye liner »* en anglais) et une poudre maquillante.

Ainsi, il est par exemple possible de mesurer un changement d'apparence visuelle d'un produit cosmétique, préférentiellement un changement de couleur d'un produit cosmétique, en contact avec de la sueur et/ou le sébum.

L'étape de détermination d'au moins un paramètre peut comprendre le contrôle de l'actuateur de pression 15 de manière à augmenter la pression dans la phase gazeuse 13 jusqu'à l'augmentation du débit dans la conduite 7 au-delà d'un seuil de débit prédéterminé. Ainsi, il est possible de détecter facilement la présence d'un composé solide formé dans la conduite 7 entre la sueur et/ou le sébum et le produit cosmétique, et/ou de mesurer sa taille, en cassant ce composé solide par une augmentation de la pression de liquide 10 entraînée par l'augmentation de la pression dans la phase gazeuse 13. Ainsi, la capacité d'un produit cosmétique à former un bouchon dans le substitut 1 peut être évaluée. Différentes compositions de produits cosmétiques peuvent ainsi être testées de manière à choisir la composition du produit cosmétique permettant de minimiser la capacité du produit cosmétique à former un bouchon.

### Utilisation du substitut pour mesurer l'efficacité d'un vernis

Un procédé de mesure de l'efficacité d'un vernis selon un aspect de l'invention peut comprendre la mesure de la perméabilité d'une couche de vernis à une solution aqueuse. Préférentiellement, un procédé de mesure la perméabilité d'un vernis recouvrant la couche cellulaire 3 et le pore 20 peut comprendre des étapes de :
- introduction de la solution aqueuse dans toute la conduite 7 en contrôlant l'actuateur de pression 15 de manière à imposer une pression dans la phase gazeuse du réservoir 12 comportant la solution aqueuse,
- mesure d'un débit de la solution aqueuse dans la conduite 7, transmission de la mesure du débit au régulateur 17, le régulateur 17 imposant une consigne de pression à l'actuateur de pression 15, de manière à imposer un débit sensiblement nul dans la conduite 7 par asservissement en boucle fermée ;
- mise en contact la solution aqueuse et du vernis à travers le pore 6,
- détermination de la perméabilité du vernis par augmentation de la pression contrôlée par l'actuateur de pression jusqu'à un débit non nul prédéterminé.

Ainsi, il est possible de tester la perméabilité et/ou l'adhérence d'un vernis en contact avec une couche cellulaire 4 au regard d'une solution aqueuse.

### Utilisation du substitut pour mesurer l'introduction de particules d'un produit

### cosmétique et/ou pharmaceutique dans un pore 6

Certains produits cosmétiques et/ou pharmaceutiques comprennent des particules, en particulier des microparticules ou des nanoparticules, c'est-à-dire des particules présentant un diamètre moyen compris entre 0,1 µm et 1 mm, et respectivement un diamètre moyen compris entre 1 nm et 1 µm. Une crème solaire peut par exemple comprendre des nanoparticules de dioxyde de titane. Il peut être utile de mesure l'introduction de telles nanoparticules dans le substitut 1 pour évaluer une éventuelle toxicité de la crème solaire appliquée sur la peau d'un être humain. Un produit cosmétique et/ou pharmaceutique dentaire peut également comprendre des microparticules destinées à boucher des pores de gencives.

Un procédé de mesure de l'introduction de particules d'un produit cosmétique et/ou pharmaceutique dans un pore 6 selon un aspect de l'invention peut comprendre les étapes de :
- introduction d'une phase liquide comprenant de la sueur naturelle ou artificielle et/ou de sébum naturel ou artificiel et/ou salive naturelle ou artificielle dans toute la conduite 7 en contrôlant l'actuateur de pression 15 de manière à imposer une pression dans la phase gazeuse du réservoir 12 comportant de la sueur et/ou du sébum,
- mesure d'un débit de la phase liquide dans la conduite 7, transmission de la mesure du débit au régulateur 17, le régulateur 17 imposant une consigne de pression à l'actuateur de pression 15, de manière à imposer un débit sensiblement nul dans la conduite 7 par asservissement en boucle fermée ;
- mise en contact de la phase liquide et du produit cosmétique et/ou pharmaceutique à travers le pore 6 et attente pendant l'introduction de particules du produit cosmétique et/ou pharmaceutique dans la phase liquide de la conduite 7, et
- détermination d'au moins un paramètre choisi parmi une concentration en particules dans la conduite 7, un paramètre physico-chimique et un paramètre biologique caractéristique de ladite réaction entre d'une part la phase liquide et d'autre part le produit cosmétique et/ou pharmaceutique de manière à mesurer l'introduction de particules d'un produit cosmétique et/ou pharmaceutique dans le pore 6.

Préférentiellement, le produit cosmétique est une crème solaire et les particules sont des particules adaptées à réfléchir la lumière du soleil, préférentiellement des particules de dioxyde de titane.

La concentration en particules dans la conduite 7 peut être mesurée par exemple par microscopie optique, par impédance dans la conduite, ou par analyse *a posteriori* de la composition de la phase liquide dans la conduite 7.

Préférentiellement, le produit pharmaceutique est un produit dentaire.

## Revendications

1. Substitut (1) de tissu corporel choisi parmi de la peau artificielle et de la muqueuse artificielle, comprenant :
- un substrat (2) comprenant une surface extérieure (3),
- une couche cellulaire (4) recouvrant au moins une partie de la surface extérieure (3), la couche cellulaire (4) comprenant une monocouche (5) de cellules de peau ou de muqueuse, présentant une épaisseur, une première face et une deuxième face (22) extérieure, opposée à la première face, la première face et la deuxième face s'étendant transversalement à l'épaisseur de la couche cellulaire (4), la couche cellulaire (4) étant disposée avec la première face en contact avec la surface extérieure (3) du substrat (2), et
- au moins un pore (6) s'étendant à travers la couche cellulaire (4), et débouchant sur la deuxième face (22) de la couche cellulaire (4),
dans lequel le substrat (2) comprend une conduite (7) propre à relier le pore (6) à un système (8) d'injection de liquide (10).

2. Substitut (1) de tissu corporel selon la revendication 1, dans lequel la couche cellulaire (4) comprend plusieurs monocouches (5) superposées.

3. Substitut (1) de tissu corporel selon la revendication 1 ou 2, dans lequel la couche cellulaire (4) comprend une partie de couche cellulaire (4) recouvrant une paroi interne de la conduite (7).

4. Substitut (1) de tissu corporel selon l'une des revendications 1 à 3, comprenant un moyen de mesure de l'état physico-chimique (9) d'un liquide (10) dans la conduite (7).

5. Substitut (1) de tissu corporel selon l'une des revendications 1 à 4, comprenant un moyen de mesure du débit d'un liquide (10) dans la conduite (7).

6. Substitut (1) de tissu corporel selon l'une des revendications 1 à 5, comprenant un moyen de mesure de l'état métabolique et/ou de l'état physico-chimique de la couche cellulaire (4).

7. Substitut (1) de tissu corporel selon l'une des revendications 1 à 6, dans lequel les cellules sont choisies au moins parmi des cellules du derme, des cellules de l'épiderme, des cellules de l'hypoderme, des cellules de la jonction dermo-épidermique, des kératinocytes tels que des kératinocytes de peau et des kératinocytes de gencive, des mélanocytes et leurs combinaisons.

8. Substitut (1) de tissu corporel selon l'une des revendications 1 à 7, comprenant en outre au moins un tensioactif (18) adsorbés sur la surface extérieure (3) du substrat (2), entre la surface extérieure (3) et la couche cellulaire (4).

9. Substitut (1) de tissu corporel selon l'une des revendications 1 à 10, dans lequel le liquide (10) est choisi au moins parmi de la sueur, de la sueur artificielle, du sébum, du sébum artificiel, de la salive, de la salive artificielle et leurs combinaisons.

10. Substitut (1) de tissu corporel selon l'une des revendications 1 à 9, dans lequel le pore présente un diamètre compris entre 0,5 µm et 3 mm, notamment compris entre 1 µm et 100 µm, et préférentiellement compris entre 3 µm et 80 µm.

11. Utilisation d'un substitut (1) de tissu corporel selon l'une des revendications 1 à 10, pour mesurer l'efficacité d'un déodorant.

12. Utilisation d'un substitut (1) de tissu corporel selon l'une des revendications 1 à 11, pour mesurer l'effet de la sueur sur un produit cosmétique recouvrant la couche cellulaire (4).

13. Utilisation d'un substitut (1) de tissu corporel selon l'une des revendications 1 à 12, pour mesurer l'effet du sébum sur un produit cosmétique recouvrant la couche cellulaire (4).

14. Utilisation d'un substitut (1) de tissu corporel selon l'une des revendications 1 à 13, pour mesurer la perméabilité d'un vernis recouvrant la couche cellulaire (4) et le pore (6).

15. Procédé de fabrication d'un substitut (1) de tissu corporel selon l'une des revendications 1 à 10, comprenant les étapes de :
- fourniture d'un substrat (2) comprenant une surface extérieure (3), et une conduite (7) propre à relier fluidiquement la surface extérieure (3) à un système d'injection de liquide (8),
- culture de cellules de peau ou de muqueuse sur la surface extérieure (3) pendant un temps prédéterminé permettant de former une couche cellulaire comprenant une monocouche (3) de cellules de peau ou de muqueuse recouvrant au moins une partie de la face extérieure (3) et comprenant également et un pore (6).

## Patentansprüche

1. Ersatz (1) für Körpergewebe, der ausgewählt ist aus künstlicher Haut und künstlicher Schleimhaut und Folgendes umfasst:
- ein Substrat (2), das eine äußere Oberfläche (3) umfasst,
- eine Zellschicht (4), die mindestens einen Teil der äußeren Oberfläche (3) bedeckt, wobei die Zellschicht (4) eine Einzelschicht (5) aus Haut- oder Schleimhautzellen umfasst, die eine Dicke, eine erste Seite und eine zweite, äußere Seite (22) aufweist, die der ersten Seite entgegengesetzt ist, wobei die erste Seite und die zweite Seite sich quer zur Dicke der Zellschicht (4) erstrecken, wobei die Zellschicht (4) mit der ersten Seite in Kontakt mit der äußeren Oberfläche (3) des Substrats (2) angeordnet ist, und
- mindestens eine Pore (6), die sich durch die Zellschicht (4) erstreckt und auf der zweiten Seite (22) der Zellschicht (4) mündet,
wobei das Substrat (2) einen Kanal (7) umfasst, der dazu geeignet ist, die Pore (6) mit einem System (8) zur Einspritzung von Flüssigkeit (10) zu verbinden.

2. Ersatz (1) für Körpergewebe nach Anspruch 1, wobei die Zellschicht (4) mehrere übereinanderliegende Einzelschichten (5) umfasst.

3. Ersatz (1) für Körpergewebe nach Anspruch 1 oder 2, wobei die Zellschicht (4) einen Zellschichtteil (4) umfasst, der eine innere Wand des Kanals (7) bedeckt.

4. Ersatz (1) für Körpergewebe nach einem der Ansprüche 1 bis 3, der ferner ein Mittel zur Messung des physikalisch-chemischen Zustands einer Flüssigkeit (10) in dem Kanal (7) umfasst.

5. Ersatz (1) für Körpergewebe nach einem der Ansprüche 1 bis 4, der ein Mittel zur Messung der Durchströmungsmenge einer Flüssigkeit (10) in dem Kanal (7) umfasst.

6. Ersatz (1) für Körpergewebe nach einem der Ansprüche 1 bis 5, der ferner ein Mittel zur Messung des metabolischen Zustands und/oder des physikalisch-chemischen Zustands der Zellschicht (4) umfasst.

7. Ersatz (1) für Körpergewebe nach einem der Ansprüche 1 bis 6, wobei die Zellen mindestens ausgewählt sind aus Zellen der Dermis, Zellen der Epidermis, Zellen der Hypodermis, Zellen der Verbindung zwischen Dermis und Hypodermis, Keratinozyten, wie beispielsweise Hautkeratinozyten und Zahnfleisch-Keratinozyten, Melanozyten und Kombinationen davon.

8. Ersatz (1) für Körpergewebe nach einem der Ansprüche 1 bis 7, der ferner mindestens einen grenzflächenaktiven Stoff (18) umfasst, der bzw. die an der äußeren Oberfläche (3) des Substrats (2) zwischen der äußeren Oberfläche (3) und der Zellschicht (4) adsorbiert ist bzw. sind.

9. Ersatz (1) für Körpergewebe nach einem der Ansprüche 1 bis 10, wobei die Flüssigkeit (10) mindestens ausgewählt ist aus Schweiß, künstlichem Schweiß, Talg, künstlichem Talg, Speichel, künstlichem Speichel und Kombinationen davon.

10. Ersatz (1) für Körpergewebe nach einem der Ansprüche 1 bis 9, wobei die Pore einen Durchmesser von zwischen 0,5 µm und 3 mm, insbesondere zwischen 1 µm und 100 µm, und vorzugsweise zwischen 3 µm und 80 µm, aufweist.

11. Verwendung eines Ersatzes (1) für Körpergewebe nach einem der Ansprüche 1 bis 10 zum Messen der Wirksamkeit eines Deodorants.

12. Verwendung des Ersatzes (1) für Körpergewebe nach einem der Ansprüche 1 bis 11 zum Messen der Wirkung von Schweiß auf ein Kosmetikprodukt, das die Zellschicht (4) bedeckt.

13. Verwendung eines Ersatzes (1) für Körpergewebe nach einem der Ansprüche 1 bis 12 zum Messen der Wirkung von Talg auf ein Kosmetikprodukt, das die Zellschicht (4) bedeckt.

14. Verwendung eines Ersatzes (1) für Körpergewebe nach einem der Ansprüche 1 bis 13 zum Messen der Durchlässigkeit eines Lacks, der die Zellschicht (4) und die Pore (6) bedeckt.

15. Verfahren zur Herstellung eines Ersatzes (1) für Körpergewebe nach einem der Ansprüche 1 bis 10, das die folgenden Schritte umfasst:
- Bereitstellen eines Substrats (2), das eine äußere Oberfläche (3) und einen Kanal (7) umfasst, der dazu geeignet ist, die äußere Oberfläche (3) fluidisch mit einem System (8) zur Einspritzung von Flüssigkeit zu verbinden,
- Kultivieren von Haut- oder Schleimhautzellen auf der äußeren Oberfläche (3) während einer vorbestimmten Zeit, die das Bilden einer Zellschicht ermöglicht, die eine Einzelschicht (3) aus Haut- oder Schleimhautzellen umfasst, die zumindest einen Teil der äußeren Seite (3) bedeckt und auch eine Pore (6) umfasst.

## Claims

1. A body tissue substitute (1) selected from artificial skin and artificial mucosa, comprising :
- a substrate (2) comprising an outer surface (3),
- a cell layer (4) covering at least part of the outer surface (3), the cell layer (4) comprising a monolayer (5) of skin or mucous membrane cells, having a thickness, a first face and a second outer face (22), opposite the first face, the first face and the second face extending transversely to the thickness of the cell layer (4), the cellular layer (4) being arranged with the first face in contact with the outer surface (3) of the substrate (2), and
- at least one pore (6) extending through the cellular layer (4) and opening onto the second face (22) of the cellular layer (4),
wherein the substrate (2) comprises a pipe (7) adapted to connect the pore (6) to a liquid injection system (8).

2. The body tissue substitute (1) according to claim 1, wherein the cell layer (4) comprises several superimposed monolayers (5).

3. A body tissue substitute (1) according to claim 1 or 2, wherein the cell layer (4) comprises a cell layer portion (4) covering an inner wall of the pipe (7).

4. A body tissue substitute (1) according to any of claims 1 to 3, comprising means for measuring the physicochemical state (9) of a liquid (10) in the pipe (7).

5. A body tissue substitute (1) according to any of claims 1 to 4, comprising means for measuring the flow rate of a liquid (10) in the pipe (7).

6. A body tissue substitute (1) according to any of claims 1 to 5, comprising means for measuring the metabolic state and/or the physicochemical state of the cell layer (4).

7. A body tissue substitute (1) according to any of claims 1 to 6, wherein the cells are selected at least from dermis cells, epidermis cells, hypodermis cells, dermal-epidermal junction cells, keratinocytes such as skin keratinocytes and gum keratinocytes, melanocytes and combinations thereof.

8. The body tissue substitute (1) according to any of claims 1 to 7, further comprising at least one surfactant (18) adsorbed on the outer surface (3) of the substrate (2), between the outer surface (3) and the cell layer (4).

9. The body tissue substitute (1) according to any of claims 1 to 10, wherein the liquid (10) is selected from at least sweat, artificial sweat, sebum, artificial sebum, saliva, artificial saliva and combinations thereof.

10. The body tissue substitute (1) according to any of claims 1 to 9, wherein the pore has a diameter between 0.5 µm and 3 mm, in particular between 1 µm and 100 µm, and preferably between 3 µm and 80 µm.

11. Use of a body tissue substitute (1) according to any of claims 1 to 10, for measuring the effectiveness of a deodorant.

12. Use of a body tissue substitute (1) according to one of claims 1 to 11, for measuring the effect of sweat on a cosmetic product covering the cell layer (4).

13. Use of a body tissue substitute (1) according to one of claims 1 to 12, for measuring the effect of sebum on a cosmetic product covering the cell layer (4).

14. Use of a body tissue substitute (1) according to any of claims 1 to 13, for measuring the permeability of a varnish covering the cell layer (4) and the pore (6).

15. A method of manufacturing a body tissue substitute (1) according to any of claims 1 to 10, comprising the steps of :
- providing a substrate (2) comprising an outer surface (3), and a pipe (7) adapted to fluidly connect the outer surface (3) to a liquid injection system (8),
- culturing skin or mucosal cells on the outer surface (3) for a predetermined time to form a cell layer comprising a monolayer (3) of skin or mucosal cells covering at least a portion of the outer surface (3) and also comprising a pore (6).
